# EUROPEAN PATENT SPECIFICATION

(11) **EP 0 519 868 B1**
(45) Date of publication and mention of the grant of the patent: **21.08.1996**
(21) Application number: 92810448.8
(22) Date of filing: 10.06.1992
(51) Int. Cl.: C07C 261/04

(54) **Preparation of dialkyl (N-cyanoimido)carbonates**
Herstellung von Dialkyl-(N-cyanoimido)carbonaten
Préparation de dialkyl-(N-cyanoimido)carbonates

(30) Priority: 19.06.1991 US 717745
(43) Date of publication of application: 23.12.1992
(73) Proprietor: CIBA-GEIGY AG, 4002 Basel (CH)
(72) Inventor: Oliver, Michael A., Baton Rouge, La. 70808 (US); Oliver, Ward H., Baton Rouge, La. 70810 (US)

(56) References cited:
- US-A- 2 708 189
- US-A- 2 819 292
- US-A- 3 225 077

## Description

This invention relates to a process for the preparation of dialkyl (N-cyanoimido)carbonates. More particularly it relates to a process for the preparation of unsubstituted or substituted dialkyl (N-cyanoimido)carbonates which comprises reacting a cyanogen hide with a dialkyl imidocarbonate in the presence of an amount of a trialkylamine or a catalytic amount of a trialkylamine and an inorganic base. It also relates to the synthesis of an unsubstituted or substituted dialkyl imidocarbonate in a non-aqueous solvent using a stoichiometric ratio (2:1) of an alcohol to cyanogen hide in the presence of an acid acceptor.

Dialkyl (N-cyanoimido)carbonates are important products which find uses as intermediates for pesticides and pharmaceuticals. Other routes to said compounds are known and are discussed for example in US patents 3,225,077 and 4,298,544 and German Offenlegungsschrift DE-OS-3225249. In general, the processes of the prior art are not satisfactory from the standpoints discussed below.

According to the prior art, a dialkyl imidocarbonate is reacted with cyanamide to give a dialkyl (N-cyanoimido)carbonate. The prior art is not satisfactory from the standpoint that it requires the use of cyanamide, an expensive and unstable reagent, and/or the process requires water or a two-phase system wherein one phase is water as the solvent. This necessitates an extraction to isolate the product.

The prior art teaching a preparation of dialkyl (N-cyanoimido)carbonates uses aqueous or mixed aqueous/water-immiscible solvent systems and a large excess of the alcohol. It has now been found that the the reaction of the dialkyl imidocarbonate with cyanogen hides is best run under nearly anhydrous conditions.

It is one aspect of the invention to prepare dialkyl (N-cyanoimido)carbonates in good yield in a non-aqueous solvent by a process which comprises either (a) adding a cyanogen halide at a controlled rate to a reactor containing an unsubstituted or substituted dialkyl imidocarbonate, an inorganic base and/or a trialkylamine, or (b) by adding a trialkylamine to a solution of the unsubstituted or substituted dialkyl imidocarbonate and a cyanogen halide.

Another aspect of the invention relates to the preparation of a dialkyl (N-cyanoimido)carbonate (I) via the preparation of a dialkyl imidocarbonate which can be used as the precursor to dialkyl (N-cyanoimido)carbonates. US patents 2,708,189 and 2,819,292 describe preparations of diallyl imidocarbonates by analogous processes, however the latter neither describes nor exemplifies the reaction.

It has also been found that the dialkyl imidocarbonate precursor can be prepared by reacting a stoichiometric ratio (2:1) of alcohol with cyanogen halide in the presence of an acid acceptor and in a non-aqueous solvent.

The use of a non-aqueous solvent system in the preparation of the dialkyl imidocarbonate is preferred, and eliminates the need to extract the product from an aqueous solution prior to the reaction with the second equivalent of the cyanogen halide. The prior art is also unsatisfactory because the excess alcohol must be removed from the system before reaction with the second equivalent of cyanogen halide to ensure maximum yields of dialkyl (N-cyanoimido)carbonate.

In addition to the reactions discussed herein, the solution of dialkyl imidocarbonate can be also used without isolation to make other products such as 1,1-dialkoxy-2,2-dicyanoethylenes and 1-alkoxy-1-amino-2,2-dicyanoethylenes, as described in Chem. Ber., 100, p.2064 (1967).

Preferred dialkyl (N-cyanoimido)carbonates are of the formula I 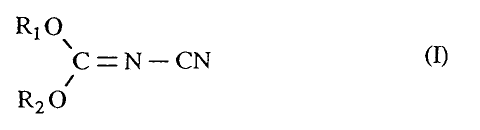 wherein
R₁ and R₂ are the same and are C₁-C₆alkyl which is unsubstituted or substituted by C₁-C₃alkoxy, phenyl or C₃-C₆cycloalkyl; or R₁ and R₂ may be part of an alkyl chain to form an optionally substituted 5 or 6 membered ring. This ring may be fused to a benzo ring system.

The alkyl groups R₁ and R₂ may be straight chain or branched. R₁ and R₂ are for example methyl, ethyl, n-propyl, isopropyl, n-butyl, sec-butyl, isobutyl and tert-butyl plus the isomeric pentyl and hexyl groups. Methyl, ethyl, propyl and isopropyl are preferred, and R₁and R₂ are the same.

C₃-C₆cycloalkyl includes cyclopropyl, cyclobutyl, cyclopentyl and cyclohexyl with cyclopropyl preferred.

C₁-C₃alkoxy includes methoxy, ethoxy, propoxy and isopropoxy.

Ring systems formed by R₁ and R₂ can be for example, 1,3-dioxolane, 1,3-dioxane, or 1,3-benzodioxolane. Suitable substituents for the 1,3-dioxane or 1,3 dioxolane rings include methyl, ethyl, propyl or isopropyl. Suitable substituents for the aromatic ring of the 1,3-benzodioxolane include C₁-C₄alkyl, C₁-C₄alkoxy or halo. 1,3-Dioxolane is preferred.

Preferred dialkyl imidocarbonates are of the formula II 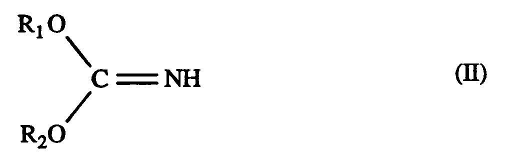 wherein R₁ and R₂ are as defined for formula I.

Preferred cyanogen hides are cyanogen chloride or cyanogen bromide. Cyanogen chloride is most preferred.

Alcohols useful in the preparation of dialkyl (N-cyanoimido)carbonates include straight or branched chain C₁-C₆alcohols such as for example methyl, ethyl, n-propyl, isopropyl, n-butyl, sec-butyl, isobutyl and tert-butyl plus the isomeric pentyl and hexyl alcohols. Methyl and ethyl alcohol are preferred. These C₁-C₆alcohols may be unsubstituted or substituted by C₁-C₃alkoxy, phenyl or C₃-C₆cycloalkyl. 1,2- and 1,3-Diols such as ethylene glycol, 1,2-and 1,3-propylene glycol, 1,2-, 1,3- and 2,3-butylene glycol and 1-phenyl-1,2-ethanediol which will form an optionally substituted 5 or 6 membered ring are also suitable alcohols. This ring may be part of a fused benzo ring system if pyrocatechol or an alkylated derivative thereof is employed as the diol.

The bases used in the preparation of dialkyl (N-cyanoimido)carbonates include trialkylamines and alkali metal carbonates with a trialkylamine catalyst. Preferred radicals for the trialkylamines include straight or branched chains of 1 to 8 carbons, wherein one or more of the carbon atoms may be replaced by an oxygen atom. The alkyl substituents of the amine may be part of ring systems, for example N-methylmorpholine and N-methylpiperidine. Preferred trialkylamines are trimethylamine and triethylamine. Preferred alkali metal carbonates are sodium carbonate and potassium carbonate. Suitable amounts of trialkylamine catalyst are 1 to 10 mole %, with 2 to 4 mole % being preferred.

If an alkali metal carbonate is used as the base in the reaction of a cyanogen hide with a dialkyl imidocarbonate, a small amount of water is necessary to activate the alkali metal carbonate. However, large amounts of water are detrimental to yields. Preferred amounts of water are 0.5 to 5 moles of water per mole of alkali metal carbonate charged, with 0.5 to 2 moles of water being particularly preferred.

Suitable solvents for the reaction of the dialkyl imidocarbonate with a cyanogen hide include open chain and cyclic ethers, halocarbons, aromatic hydrocarbons, halogenated aliphatic and aromatic hydrocarbons, ketones and esters. Ethers are the most preferred solvents. Other preferred solvents include C₃-C₇ketones, C₁-C₆hydrocarbons substituted by 1-6 halogens, and benzene and phenyls substituted by 1-2 halogens or C₁-C₃alkyl groups. Preferred individual solvents include for example, diethylether, tert-butyl methylether, tetrahydrofuran, dioxane, methylene chloride, toluene and acetone. Most preferred are t-butyl methyl ether, tetrahydrofuran, acetone or methylene chloride.

Temperatures for the reaction of a cyanogen halide with the dialkyl imidocarbonate can range from -20 to 40°C, preferably from about -10 to 10°C.

For the reaction of a cyanogen hide with a dialkyl imidocarbonate, modes of addition include adding the cyanogen halide to a solution of the dialkyl imidocarbonate and base, adding the base to a solution of the dialkyl imidocarbonate and cyanogen hide, or concurrent addition of the dialkyl imidocarbonate and base to a solution of cyanogen hide. Adding the cyanogen halide to a solution of the dialkyl imidocarbonate and base is preferred.

The other aspect of the invention relates to the preparation of unsubstituted or substituted dialkyl imidocarbonates which comprises reacting a stoichiometric ratio (2:1) of an alcohol with a cyanogen halide in the presence of an acid acceptor and in a non-aqueous solvent.

Accordingly a process is described for the preparation of dialkyl imidocarbonates of the formula II 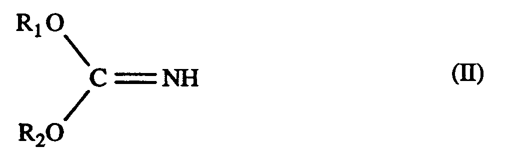
wherein R₁ and R₂ are as defined for formula I, and have the same preferred aspects as given for formula I.

Preferred alcohols and cyanogen hides used in the preparation of dialkyl imidocarbonates of formula II are as described above for the preparation of dialkyl (N-cyanoimido)carbonates of formula I.

Suitable acid acceptors for the preparation of the dialkyl imidocarbonates are alkali metal hydroxides or alkali metal alkoxides having the same alkyl moiety as in the desired dialkyl imidocarbonate. If the dialkyl imidocarbonate solution is to be isolated and an alkali metal hydroxide is used as the acid acceptor, two equivalents of the hydroxide should be used. The second equivalent will adsorb the water generated in the reaction and the resulting salt/hydroxide slurry can then be removed by filtration. The solution of dialkyl imidocarbonate thus obtained can be used in subsequent reactions. Preferred alkali metal hydroxides are sodium hydroxide or potassium hydroxide. Preferred alkali metal alkoxides include sodium methoxide and sodium ethoxide.

Suitable solvents for the reaction to form the dialkyl imidocarbonate are ethers, halocarbons, halogenated hydrocarbons and aromatic hydrocarbons, for example, diethylether, tert-butyl methylether, tetrahydrofuran, dioxane, methylene chloride or toluene. The preferred solvents are tert-butyl methylether, tetrahydrofuran, methylene chloride or toluene.

Temperatures for the reaction to form the dialkyl imidocarbonate can range from - 20 to 20°C, preferably from about -10 to 5°C.

The two reactions can be run in sequence in a single reactor if one equivalent of alkali metal hydroxide or alkali metal alkoxide, and one to two equivalents of an alkali metal carbonate are used as the bases. The carbonate can be added at the beginning of the first cyanogen halide addition or prior to the second cyanogen halide addition. The trialkalamine catalyst should not be added before the formation of the dialkyl imidocarbonate is complete. A minimum hold time of two hours at 0°C is required before the addition of the catalyst and the second equivalent of the cyanogen halide.

The preferred method for carrying out the reactions in sequence is to slurry one equivalent of sodium hydroxide and one and a half equivalents of sodium carbonate in an ether solvent. Two equivalents of an alcohol, e.g. methanol, ethanol, n-propanol or isopropanol, are added while cooling the mixture to about 0°C. Cyanogen chloride is then added over about a four hour period while maintaining a reaction temperature of 0-5°C. The reaction mixture is held at 0-5°C for two hours while the reaction to form the dialkyl imidocarbonate is completed. After the hold period is complete, 2-4 mole % of a trialkylamine, e.g. trimethylamine, and a molar equivalent of water are added. The second equivalent of cyanogen chloride is then added over a 2-4 hour period while maintaining a temperature of 0-5°C. After an optional hold period, the reaction is filtered to remove the salts and the solvent evaporated to yield the crude dialkyl (N-cyanoimido)carbonate.

The invention is illustrated but not limited by the following examples. Cyanogen chloride solutions in tert-butyl methylether (MTBE) used for most examples were prepared in the laboratory and typically contained 1-2 % water. Therefore unless otherwise specified, no additional water was added to the reaction mixture when cyanogen chloride from this source was used.

### Example 1. Dimethyl (N-cyanoimido)carbonate

To a 500 ml reactor are added 150 ml tert-butyl methylether (MTBE) and 25.3 g (0.633 mole) sodium hydroxide in bead form. The reactor contents are cooled to 0°C and 20.5 g of methanol (0.639 mole) is charged while maintaining the reaction temperature at 0 to 5°C. Then 19.4 g gaseous cyanogen chloride is charged over 1.5 hours while maintaining the temperature at -5 to 0°C. The reaction mixture is held at 0°C for an additional two hours. At the end of the hold period, 37.0 g sodium carbonate, 1.5 g trimethylamine (25% in water, 7 mmol), and 3.5 g water are added to the reaction mixture. 20.5 g cyanogen chloride is charged over 1.5 hours while maintaining the temperature at -5 to 0°C. The reaction mixture is held at 0°C for one hour then warmed to 25°C. The reaction mixture is filtered and the solvent is evaporated from the filtrate under vacuum. The salt cake is washed twice with 50 ml methylene chloride and the filtrate is added to the crude product. The methylene chloride is evaporated under vacuum to obtain 23.1 g dimethyl (N-cyanoimido)carbonate. Assay 95.2 %, (61 % yield).

### Example 2. Dimethyl (N-cyanoimido)carbonate

To a 500 ml reactor are added 50 ml tert-butyl methylether (MTBE) and 44.0 g (1.0 mole) sodium hydroxide in bead form. The reactor contents are cooled to 0°C and 32.0 g methanol (1.00 mole) is charged while maintaining the reaction at 0 to 5°C. 100g cyanogen chloride solution (31.0% in MTBE, 0.50 mole) is added to the stirred reaction mixture over 30 minutes while maintaining the temperature at 0 to 5°. After the addition is complete, the reaction mixture is held at -5 to 0°C overnight. The reaction mixture is filtered and then the salt cake is washed with 50 ml MTBE. The combined filtrates are added to a clean 1 liter flask along with 58.5 g sodium carbonate, 3.6 g trimethylamine (25% in water, 10 mmol), and 5.4 g water. The reactor contents are cooled to -5°C and 106 g (31% in MTBE, 0,53 mole) cyanogen chloride solution is charged over 5 hours while maintaining the reaction mixture at 0 to 5°C. After the addition is complete, the reaction mixture is held for two hours at 0 to 5°C. The reaction mixture is then filtered and the cake is washed with 75 ml MTBE. The filtrates are combined and the solvent is removed under vacuum to yield 28.3 g dimethyl (N-cyanoimido)carbonate, 90% assay (45 % yield).

### Example 3. Dimethyl (N-cyanoimido)carbonate

To a 500 ml reactor are added 50 ml tert-butyl methylether (MTBE) and 40.0 g ( 1.0 mole) sodium hydroxide in bead form. The reactor contents are cooled to 0°C and 32.1 g methanol (1.00 mole) is added while maintaining the reaction at 0 to 5°C. Then 104g cyanogen chloride solution (29.4% in MTBE, 0.50 mole) is added to the stirred reaction mixture over 1.5 hours while maintaining the temperature at 0 to 5°C. After the addition is complete, the mixture is stirred at 0°C for an additional 2 hours. At the end of the holding period the reaction mixture is filtered and the salts are washed with 50 ml MTBE. The filtrates are combined, added to a clean flask and cooled to 0 to 5°C. Then 102.1 g a cyanogen chloride solution (29.4% in MTBE, 0.49 mole) is added over 15 minutes. Maintaining the temperature at 0 to 5°C, 30 g triethylamine (97%, water 2%) is added over 2.5 hours. When the addition is complete, the reaction mixture is filtered. Then the salt cake is washed three times with 75 ml boiling MTBE. The solvent is removed under vacuum. Then the crystals are washed twice with 15 ml ice cold MTBE. The product is dried under vacuum to obtain 23.2 g dimethyl (N-cyanoimido)carbonate, assay 93.9 % (38 % yield).

### Example 4. Diethyl (N-cyanoimido)carbonate

To a 1 liter reactor are added 50 ml tert-butyl methylether, 40 g (1.0 mole) sodium hydroxide (beads) and 48.5 g 95% ethanol (1.0 mole). The reactor contents are cooled to - 5°C and 103 g cyanogen chloride solution (30% in MTBE, 0.50 mole) is added over 2 hours while stirring, maintaining the reaction mixture at -5 to 0°C. After the addition is complete, the mixture is stirred at 0 to 10°C for an additional two hours. At the end of the period, the reaction mixture is filtered and the salt cake is washed with 50 ml MTBE. The combined filtrates are added to a clean 1 liter flask along with 58.3 g sodium carbonate and 2.36 g trimethylamine (25% in water, 10 mmol). The reactor contents are cooled to -5°C and 103 g cyanogen chloride solution (30% in MTBE, 0.50 mole) is added over 1 hour while maintaining the reaction mixture at 0 to 5°C. After the addition is complete, the mixture is stirred for one hour at 0 to 5°C, then warmed to 25°C and held for an additional hour. The reaction mixture is filtered and the solvent is removed under vacuum to obtain 51.2 g diethyl (N-cyanoimido)carbonate, 82.5% assay (59.5 % yield).

### Example 5. Di-n-propyl (N-cyanoimido)carbonate

To a 1 liter reactor are added 50 ml tert-butyl methylether, 40 g (1.0 mole) sodium hydroxide (beads) and 60.1 g n-propanol (1.0 mole). The reactor contents are cooled to 0°C and 96 g cyanogen chloride solution is added (32.6% in MTBE, 0.50 mole) over 1.5 hours while maintaining the reaction temperature at 0 to 5°C. After the addition is complete, the reaction mixture is stirred at 0 to 5°C for an additional two hours. At the end of the hold period, the reaction mixture is filtered and the saltcake is washed with 50 ml MTBE. The combined filtrates are added to a clean 1 liter flask along with 58.3 g sodium carbonate and 2.3 g trimethylamine (25% in water, 10 mmol). The reactor contents are cooled to - 5°C and 96 g cyanogen chloride solution is added (32.6% in MTBE, 0.50 mole) over 1.5 hours while maintaining the reaction mixture at -5 to 5°C. After the addition is complete, the reaction mixture is stirred for one hour while allowing the temperature to slowly reach 20°C. The reaction mixture is then filtered and the solids are washed with 75 ml MTBE. The filtrates are combined and the solvent is removed under vacuum to yield 68.5 g di-n-propyl (N-cyanoimido)carbonate, 80.0% assay (64.5 % yield).

### Example 6. Diisopropyl (N-cyanoimido)carbonate

To a 1 liter reactor are added 50 ml tert-butyl methyl ether, 40 g (1.0 mole) sodium hydroxide (beads) and 60.1 g isopropanol (1.0 mole). The reactor contents are cooled to 0°C and 106 g cyanogen chloride solution (29% in MTBE, 0.50 mole) is charged over 2.5 hours while maintaining the reaction at -5 to 5°C. After the addition is complete, the reaction mixture is stirred at -5 to 5°C for an additional two hours. At the end of the holding period the reaction mixture is filtered and the saltcake is washed with 75 ml MTBE. The combined filtrates are added to a clean 1 liter flask along with 58.3 g sodium carbonate and 2.36 g trimethylamine (25% in water, 10 mmol). The reactor contents are cooled to -5°C and 106 g cyanogen chloride solution (29% in MTBE, 0.50 mole) is charged over 1.5 hours while maintaining the reaction mixture at -5 to 5°C. After the addition is complete, the reaction mixture is allowed to warm to 25°C. The reaction mixture is filtered and the solvent is removed under vacuum to yield 50.6 g diisopropyl (N-cyanoimido)carbonate, 90% assay (53.6 % yield).

## Claims

1. A process for the preparation of unsubstituted or substituted dialkyl (N-cyanoimido)carbonates which comprises reacting a cyanogen halide with an unsubstituted or substituted dialkyl imidocarbonate in the presence of a trialkylamine or a catalytic amount of a trialkylamine and an inorganic base, in a non-aqueous solvent.

2. A process according to claim 1, wherein the dialkyl (N-cyanoimido)carbonate is of the formula I 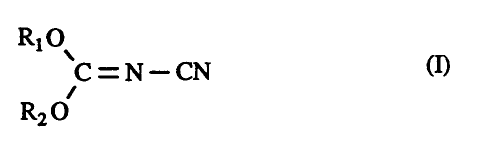 wherein
R₁ and R₂ are the same and are C₁-C₆alkyl which is unsubstituted or optionally substituted by C₁-C₃alkoxy, phenyl or C₃-C₆cycloalkyl; or
R₁ and R₂ may be part of an alkyl chain which forms an optionally substituted 5 or 6 membered ring, which ring may be fused to a benzo ring system.

3. A process according to claim 2, wherein R₁ and R₂ are the same and are methyl, ethyl, propyl or isopropyl.

4. A process according to claim 1, wherein the cyanogen halide is cyanogen chloride or cyanogen bromide.

5. A process according to claim 1, wherein the inorganic base is an alkali metal carbonate.

6. A process according to claim 1, wherein the trialkylamine is a tri(C₁-C₈)alkylamine, and one or more of the carbon atoms may be replaced by an oxygen atom.

7. A process according to claim 6, wherein the trialkylamine is trimethylamine or triethylamine.

8. A process according to claim 5 wherein 1 to 10 mole% of a trialkyl amine is used in addition to an alkali metal cabonate.

9. A process according to claim 8, wherein 2 to 4 mole % of triethyl- or trimethylamine is used, and the alkali metal carbonate is sodium carbonate or potassium carbonate.

10. A process according to claim 1, which is conducted in a non-aqueous solvent.

11. A process according to claim 10, wherein said solvent is an open chain or cyclic ether, a C₃-C₇ketone, a C₁-C₆hydrocarbon substituted with 1 to 6 halogens, benzene or a phenyl substituted with 1 or 2 halogens or C₁-C₃alkyl groups, or a mixture thereof.

12. A process according to claim 11, wherein the solvent is t-butyl methylether, tetrahydrofuran, acetone, or methylene chloride.

13. A process according to claim 1, wherein the temperature is in the range of -20 to 40°C.

14. A process according to claim 13, wherein the temperature is in the range of -10 to 10°C.

15. A process according to claim 1, which comprises adding a cyanogen halide to a solution of the dialkyl imidocarbonate and base, adding the base to a solution of the dialkyl imidocarbonate and cyanogen halide, or concurrent addition of the dialkyl imidocarbonate and base to a solution of cyanogen halide.

16. A process according to claim 15, which comprises adding the cyanogen chloride to a solution of the dialkyl imidocarbonate and base.

17. A process according to claim 1, in which the dialkyl imidocarbonate is prepared by reacting a stoichiometric ratio (2:1) of an alcohol with a cyanogen halide in the presence of an acid acceptor and in a non-aqueous solvent.

18. A process according to claim 17, wherein the dialkyl imidocarbonate is of the formula II 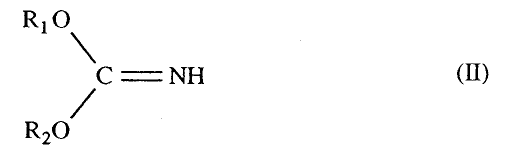 wherein
R₁ and R₂ are the same and are C₁-C₆alkyl which is unsubstituted or optionally substituted by C₁-C₃alkoxy phenyl or C₃-C₆cycloalkyl; or
R₁ and R₂ may be part of an alkyl chain which forms an optionally substituted 5 to 6 membered ring, which ring may be fused to a benzo ring system.

19. A process according to claim 18, wherein R₁ and R₂ are the same and are methyl, ethyl, propyl or isopropyl.

20. A process according to claim 17, wherein the cyanogen halide is cyanogen chloride or cyanogen bromide.

21. A process according to claim 17, wherein the acid acceptor is a alkali metal hydroxide or an alkali metal alkoxide with the same alkyl moiety as in the desired dialkyl imidocarbonate.

22. A process according to claim 17, wherein the acid acceptor is sodium hydroxide or potassium hydroxide.

23. A process according to claim 17, wherein the solvent is an ether, halocarbon, halogenated hydrocarbon, or aromatic hydrocarbon.

24. A process according to claim 23, wherein the solvent is t-butyl methylether, tetrahydrofuran, methylene chloride or toluene.

25. A process according to claim 17, wherein the reaction temperature is in the range from -20 to 20°C.

26. A process according to claim 25, wherein the reaction temperature is in the range from - 10 to 5°C.

27. A process according to claim 1, which comprises slurrying one equivalent of sodium hydroxide and one and a half equivalents of sodium carbonate in t-butyl methylether, adding two equivalents of an alcohol, cooling the mixture to about 0°C, adding cyanogen chloride over about a four hour period while maintaining a reaction temperature in the range of 0-5°C, holding the reaction mixture in the same temperature range until the reaction to form the dialkyl imidocarbonate is essentially complete, adding 2-4 mole % of a trialkylamine, 1 molar equivalent of water, adding a second equivalent of cyanogen chloride over a 2-4 hour period while maintaining a temperature of 0-5°C, stirring until the reaction is essentially complete, filtering the mixture to remove salts and then evaporating the solvent to yield a crude dialkyl (N-cyanoimido)carbonate.

28. A process according to claim 27, wherein the alcohol is methanol, ethanol, n-propanol or isopropanol.

29. A process according to claim 27, wherein the trialkylamine is trimethylamine.

## Patentansprüche

1. Verfahren zur Herstellung von unsubstituierten oder substituierten Dialkyl(N-cyanoimido)carbonaten, umfassend Umsetzen eines Halogencyans mit einem unsubstituierten oder substituierten Dialkylimidocarbonat in Gegenwart eines Trialkylamins oder einer katalytischen Menge eines Trialkylamins und einer anorganischen Base in einem nichtwässerigen Lösungsmittel.

2. Verfahren nach Anspruch 1, wobei das Dialkyl(N-cyanoimido)carbonat die Formel I 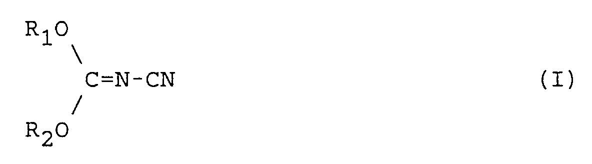 aufweist, worin R₁ und R₂ gleich sind und C₁-C₆-Alkyl, unsubstituiert oder gegebenenfalls mit C₁-C₃-Alkoxy, Phenyl oder C₃-C₆-Cycloalkyl substituiert, darstellen; oder R₁ und R₂ Teil einer Alkylkette sein können, die einen gegebenenfalls substituierten 5- oder 6-gliedrigen Ring bildet, wobei der Ring an ein Benzoringsystem kondensiert sein kann.

3. Verfahren nach Anspruch 2, wobei R₁ und R₂ gleich sind und Methyl, Ethyl, Propyl oder Isopropyl darstellen.

4. Verfahren nach Anspruch 1, wobei das Halogencyan Chlorcyan oder Bromcyan ist.

5. Verfahren nach Anspruch 1, wobei die anorganische Base ein Alkalimetallcarbonat darstellt.

6. Verfahren nach Anspruch 1, wobei das Trialkylamin ein Tri(C₁-C₈)alkylamin darstellt und ein oder mehrere der Kohlenstoffatome durch Sauerstoff ersetzt sein können.

7. Verfahren nach Anspruch 6, wobei das Trialkylamin Trimethylamin oder Triethylamin darstellt.

8. Verfahren nach Anspruch 5, wobei 1 bis 10 Mol-% eines Trialkylamins zusätzlich zu einem Alkalimetallcarbonat verwendet werden.

9. Verfahren nach Anspruch 8, wobei 2 bis 4 Mol-% Triethyl- oder Trimethylamin verwendet werden und das Alkalimetallcarbonat Natriumcarbonat oder Kaliumcarbonat ist.

10. Verfahren nach Anspruch 1, das in einen nichtwässerigen Lösungsmittel durchgeführt wird.

11. Verfahren nach Anspruch 10, wobei das Lösungsmittel einen offenkettigen oder cyclischen Ether, ein C₃-C₇-Keton, einen C₁-C₆-Kohlenwasserstoff, substituiert mit 1 bis 6 Halogenatomen, Benzol oder ein Phenyl, substituiert mit 1 oder 2 Halogenatomen oder C₁-C₃-Alkylgruppen, oder ein Gemisch davon darstellt.

12. Verfahren nach Anspruch 11, wobei das Lösungsmittel t-Butylmethylether, Tetrahydrofuran, Aceton oder Methylenchlorid darstellt.

13. Verfahren nach Anspruch 1, wobei die Temperatur im Bereich von -20 bis 40°C liegt.

14. Verfahren nach Anspruch 13, wobei die Temperatur im Bereich von -10 bis 10°C liegt.

15. Verfahren nach Anspruch 1, umfassend Zugeben des Halogencyans zu einer Lösung des Dialkylimidocarbonats und der Base, Zugabe der Base zu einer Lösung des Dialkylimidocarbonats und Halogencyans oder gleichzeitige Zugabe des Dialkylimidocarbonats und der Base zu einer Lösung des Halogencyans.

16. Verfahren nach Anspruch 15, umfassend Zugabe des Chlorcyans zu einer Lösung des Dialkylimidocarbonats und der Base.

17. Verfahren nach Anspruch 1, wobei das Dialkylimidocarbonat durch Umsetzen eines stöchiometrischen Verhältnisses (2:1) eines Alkohols mit einem Halogencyan in Gegenwart eines Säureakzeptors und in einem nichtwässerigen Lösungsmittel hergestellt wird.

18. Verfahren nach Anspruch 17, wobei das Dialkylimidocarbonat die Formel II 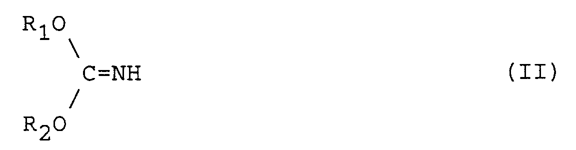 aufweist, worin R₁ und R₂ gleich sind und C₁-C₆-Alkyl, unsubstituiert oder gegebenenfalls mit C₁-C₃-Alkoxy, Phenyl oder C₃-C₆-Cycloalkyl substituiert, darstellen; oder R₁ und R₂ Teil einer Alkylkette sein können, die einen gegebenenfalls substituierten 5- bis 6-gliedrigen Ring bildet, wobei der Ring an ein Benzoringsystem kondensiert sein kann.

19. Verfahren nach Anspruch 18, wobei R₁ und R₂ gleich sind und Methyl, Ethyl, Propyl oder Isopropyl darstellen.

20. Verfahren nach Anspruch 17, wobei das Halogencyan Chlorcyan oder Bromcyan darstellt.

21. Verfahren nach Anspruch 17, wobei der Säureakzeptor ein Alkalimetallhydroxid oder Alkalimetallalkoxid mit dem gleichen Alkylrest wie in dem gewünschten Dialkylimidocarbonat darstellt.

22. Verfahren nach Anspruch 17, wobei der Säureakzeptor Natriumhydroxid oder Kaliumhydroxid ist.

23. Verfahren nach Anspruch 17, wobei das Lösungsmittel ein Ether, Halogenkohlenstoff, halogenierter Kohlenwasserstoff oder aromatischer Kohlenwasserstoff ist.

24. Verfahren nach Anspruch 23, wobei das Lösungsmittel t-Butylmethylether, Tetrahydrofuran, Methylenchlorid oder Toluol ist.

25. Verfahren nach Anspruch 17, wobei die Umsetzungstemperatur im Bereich von -20 bis 20°C liegt.

26. Verfahren nach Anspruch 25, wobei die Reaktionstemperatur im Bereich von -10 bis 5°C liegt.

27. Verfahren nach Anspruch 1, umfassend Aufschlämmen eines Äquivalents Natriumhydroxid und anderthalb Äquivalente Natriumcarbonat in t-Butylmethylether, Zugabe von zwei Äquivalenten eines Alkohols, Abkühlen des Gemisches auf etwa 0°C, Zugabe von Chlorcyan über einen Zeitraum von etwa vier Stunden, während Halten der Reaktionstemperatur im Bereich von 0-5°C, Halten der Temperatur des Reaktionsgemisches in dem gleichen Temperaturbereich, bis die Reaktion zur Bildung des Dialkylimidocarbonats im wesentlichen beendet ist, Zugabe von 2-4 Mol-% eines Trialkylamins, 1 Moläquivalent Wasser, Zugabe eines zweiten Äquivalents Chlorcyan über einen Zeitraum von 2-4-Stunden, während Halten einer Temperatur von 0-5°C, Rühren, bis die Reaktion im wesentlichen beendet ist, Filtrieren des Gemisches zur Entfernung von Salzen und anschließend Verdampfen des Lösungsmittels unter Gewinnung eines rohen Dialkyl(N-cyanoimido)carbonats.

28. Verfahren nach Anspruch 27, wobei der Alkohol Methanol, Ethanol, n-Propanol oder Isopropanol ist.

29. Verfahren nach Anspruch 27, wobei das Trialkylamin Trimethylamin darstellt.

## Revendications

1. Procédé de préparation de (N-cyanoimido)-carbonates de dialkyle substitués ou non substitués qui comprend le fait de faire réagir un halogénure de cyanogène avec un imidocarbonate de dialkyle substitué ou non substitué en présence d'une trialkylamine ou d'une quantité catalytique de trialkylamine et d'une base minérale dans un solvant non aqueux.

2. Procédé selon la revendication 1, où le (N-cyanoimido)-carbonate de dialkyle est de formule I 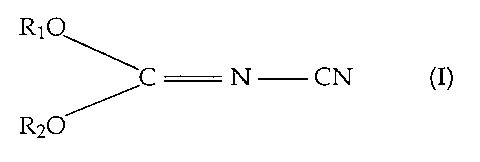 où
R₁ et R₂ sont identiques et représentent un alkyle
C₁₋₆ qui est non substitué ou substitué éventuellement par un alcoxy C₁₋₃, un phényle ou un cycloalkyle C₃₋₆;
ou
R₁ et R₂ peuvent faire partie d'une chaîne alkyle qui forme un cycle à 5 ou 6 maillons, éventuellement substitué, ce cycle petit être fusionné pour donner un système cyclique benzoïque.

3. Procédé selon la revendication 2, où R₁ et R₂ sont identiques et représentent un méthyle, un éthyle, un propyle ou un isopropyle.

4. Procédé selon la revendication 1, où l'halogénure de cyanogène est le chlorure de cyanogène ou le bromure de cyanogène.

5. Procédé selon la revendication 1, où la base minérale est un carbonate de métal alcalin.

6. Procédé selon la revendication 1, où la trialkylamine est une trialkylamine C₁₋₈, et un ou plusieurs des atomes de carbone peuvent être remplacés par un atome d'oxygène.

7. Procédé selon la revendication 6, où la trialkylamine est la triméthylamine ou la triéthylamine.

8. Procédé selon la revendication 5, où 1 à 10 % molaire d'une trialkylamine sont utilisés en plus d'un carbonate de métal alcalin.

9. Procédé selon la revendication 8, où 2 à 4 % molaire de triéthyl- ou triméthylamine sont utilisés, et le carbonate de métal alcalin est du carbonate de sodium ou du carbonate de potassium.

10. Procédé selon la revendication 1, qui est réalisé dans un solvant non aqueux.

11. Procédé selon la revendication 10, où ledit solvant est un éther cyclique ou à chaîne ouverte, une cétone C₃₋₇, un hydrocarbure C₁₋₆ substitué par 1 à 6 atomes d'halogène, le benzène, ou un phényle substitué par un ou deux atomes d'halogène ou des groupes alkyle C₁₋₃, ou un mélange de ces solvants.

12. Procédé selon la revendication 11, où le solvant est l'éther t-butylméthylique, le tétrahydrofuranne, l'acétone ou le chlorure de méthylène.

13. Procédé selon la revendication 1, où la température est comprise dans l'intervalle de -20 à 40°C.

14. Procédé selon la revendication 13, où la température est dans l'intervalle de -10 à 10°C.

15. Procédé selon la revendication 1, qui comprend le fait d'ajouter un halogénure de cyanogène à une solution d'imidocarbonate de dialkyle et de base, d'ajouter la base à une solution d'imidocarbonate de dialkyle et d'halogénure de cyanogène, ou de réaliser l'addition concurrente de l'imidocarbonate de dialkyle et de base à une solution d'halogénure de cyanogène.

16. Procédé selon la revendication 15, qui comprend le fait d'ajouter le chlorure de cyanogène à une solution d'imidocarbonate de dialkyle et de base.

17. Procédé selon la revendication 1, où l'imidocarbonate de dialkyle est préparé en faisant réagir un rapport stoechiométrique (2:1) d'un alcool avec un halogénure de cyanogène en présence d'un accepteur d'acide dans un solvant non aqueux.

18. Procédé selon la revendication 17, où l'imidocarbonate de dialkyle est de formule II 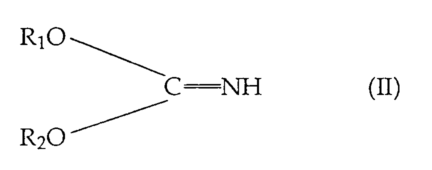 où
R₁ et R₂ sont identiques et représentent un alkyle
C₁₋₆ qui est non substitué ou éventuellement substitué par un alcoxy C₁₋₃, un phényle ou un cycloalkyle C₃₋₆ ;
ou
R₁ et R₂ peuvent faire partie d'une chaîne alkyle qui forme un cycle de 5 à 6 maillons éventuellement substitué, ce cycle peut être fusionné pour former un système cyclique benzoïque.

19. Procédé selon la revendication 18, où R₁ et R₂ sont identiques, et représentent un méthyle, éthyle, propyle ou isopropyle

20. Procédé selon la revendication 17, où l'halogénure de cyanogène est le chlorure de cyanogène ou le bromure de cyanogène.

21. Procédé selon la revendication 17, où l'accepteur d'acide est un hydroxyde de métal alcalin ou un alcoxyde de métal alcalin avec le même radical alkyle que celui souhaité dans l'imidocarbonate de dialkyle.

22. Procédé selon la revendication 17, où l'accepteur d'acide est l'hydroxyde de sodium ou l'hydroxyde de potassium.

23. Procédé selon la revendication 17, où le solvant est un éther, un halocarbone, un hydrocarbure halogéné ou un hydrocarbure aromatique.

24. Procédé selon la revendication 23, où le solvant est l'éther t-butylméthylique, le tétrahydrofuranne, le chlorure de méthylène ou le toluène.

25. Procédé selon la revendication 17, où la température de réaction est dans l'intervalle de -20 à 20°C.

26. Procédé selon la revendication 25, où la température de réaction est dans l'intervalle de -10 à 5°C.

27. Procédé selon la revendication 1, qui comprend le fait d'empâter un équivalent d'hydroxyde de sodium et un équivalent et demi de carbonate de sodium dans l'éther t-butyl-méthylique, d'ajouter deux équivalents d'un alcool, de refroidir le mélange à environ 0°C, d'ajouter du chlorure de cyanogène en une période d'environ quatre heures, tout en maintenant la température de réaction dans l'intervalle de 0 à 5°C, de maintenir le mélange réactionnel dans le même intervalle de température jusqu'à ce que la réaction de formation de l'imidocarbonate de dialkyle soit sensiblement complète, d'ajouter 2-4 % molaire d'une trialkylamine, un équivalent molaire d'eau, d'ajouter un second équivalent de chlorure de cyanogène en une période de 2-4 heures, tout en maintenant la température entre 0 et 5°C, d'agiter jusqu'à ce que la réaction soit essentiellement complète, de filtrer le mélange réactionnel pour retenir les sels, et ensuite d'évaporer le solvant pour donner un (N-cyanoimido)-carbonate de dialkyle brut.

28. Procédé selon la revendication 27, où l'alcool est le méthanol, l'éthanol, le n-propanol ou l'isopropanol.

29. Procédé selon la revendication 27, où la trialkylamine est la triméthylamine.
